# EUROPEAN PATENT SPECIFICATION

(11) **EP 1 092 515 B1**
(45) Date of publication and mention of the grant of the patent: **21.05.2003**
(21) Application number: 00121449.3
(22) Date of filing: 29.09.2000
(51) Int. Cl.: B26B 9/00, B21D 53/64, A61B 17/32, B26B 21/54

(54) **Methods for making atomically sharp edged cutting blades.**
Verfahren zur Herstellung atomar scharfer Schneidklingen.
Procédés pour la fabrication de lâmes tranchantes à acuité atomaire.

(30) Priority: 15.10.1999 US 159678 P; 10.08.2000 US 635908
(43) Date of publication of application: 18.04.2001
(73) Proprietor: NEWMAN, Martin H., Sharon, MA 02067 (US)
(72) Inventor: NEWMAN, Martin H., Sharon, MA 02067 (US)
(74) Representative: Prüfer, Lutz H., Dipl.-Phys.

(56) References cited:
- US-A- 4 933 058
- US-A- 4 980 021
- US-A- 5 032 243

## Description

### FIELD OF THE INVENTION

The present invention relates to devices having extremely sharp cutting edges, which are particularly useful for surgical instruments, and methods of making said devices. Indeed, the invention relates to a process for forming an atomically sharp cutting edge in a material using single or dual focused ion beam milling and the devices produced thereby. The invention is particularly useful in the manufacture of surgical cutting instruments, yet can be used to provide almost any cutting edge where increased sharpness of the cutting edge is desirable.

### BACKGROUND OF THE INVENTION

Modern medical procedures require cutting instruments of exceptional sharpness and wear resistance and which, moreover, exhibit minimal tissue resistive forces. In delicate microsurgery and especially ophthalmologic surgery, cutting edges must be extremely sharp and must maintain that sharpness throughout the operation. However, even presently available acutely sharpened blades can exhibit substantial resistive forces, making it difficult to move through tissue without producing a "ragged" cut. Moreover, studies have shown that blade degradation can lead to tissue damage, post-operative complications, and slower healing.

A critical element of a surgical blade is the cutting edge. Chips, nicks or breaks in the integrity of the edge, residual burrs, and/or rolled or distorted cutting edges of the blade can render the blade useless or, even worse, can injure the patient.

Sharp-edged cutting instruments typically are produced from metals such as stainless steel, Carborundum, or other relatively hard materials, such as silicon carbide, silicon, glass, sapphires, rubies or diamonds. Glass, silicon and stainless steel are relatively cheap and therefore disposable, while diamonds, rubies and sapphires are relatively expensive and, of necessity, typically require reuse as a matter of economics. Each of these materials can be ground, stamped, etched, lapped or honed by a myriad of means to provide a cutting edge. For example, metal can be ground, stamped and/or etched to produce cutting blades with extremely fine cutting edges. However, the thinner the cutting edge of the metal becomes, the narrower becomes the bevel angle(s) that forms that cutting edge. As a result, thinner-edge metal cutting blades exhibit greater fragility than relatively thicker edged blades. This fragility manifests by significant wear, i.e., chips, nicks, breaks, residual burrs, and/or rolled or distorted cutting edges. Moreover, metal cutting blades can dull significantly even during a single use.

Many of those skilled in the art have considered a diamond blade as the accepted standard for sharpness. However, diamond blades are very expensive, extremely delicate, and still require resharpening on a regular basis. Thus, those skilled in the art have sought, by a variety of means, more economical means of fashioning cutting devices with diamond-like sharpness. Some of the more recent attempts to provide hard sharp cutting edges are discussed below.

Henderson (US 4,534,827) discloses a cutting instrument fabricated by etching and chemically polishing a single crystal of aluminum oxide material, e.g., rubies or sapphires, to form an edge having a maximum radius of curvature of about 100 Angstroms (Å). However, the disclosed materials are brittle and, moreover, the cutting blades formed by the lattice of the material exhibit a natural bevel incline.

Mirtich et al. (US 4,490,229) discloses a method for making diamond like carbon films on a substrate. The surface of the substrate is exposed to an argon ion beam that contains hydrocarbon. At the same time, a second argon ion beam (without hydrocarbon) having greater ion energy is directed toward the surface, which increases mobility of the condensing atoms and removes lesser bound atoms.

Bache et al. (US 4,933,058) discloses a method for coating a cutting substrate with a harder material by chemical vapor deposition or sputtering, while simultaneously subjecting the cutting edge to ion bombardment. Ion bombardment causes preferred depositional orientation of the harder material and, moreover, causes sputter removal of the deposited material, which produces a coating with a particular cross-sectional shape and ultimate tip radius.

Kokai (Japanese PN 61-210179) discloses the application of coatings of amorphous carbon (silicon carbide) by plasma-induced vapor-phase deposition in a gaseous mixture of hydrogen and hydrogen compounds (e.g., methane) to produce a cutting edge with a thickness between 1 nm and 20 nm.

Hoshino (US 4,832,979) describes a process for preparing a laser knife wherein the surface of a probe portion of the knife is coated with a carbon coating of 1 to 50 µm thick, on which is coated a 1 to 50 µm thick protective coating of sapphire, ruby or quartz glass.

Kitamura et al. (US 4,839,195) discloses forming a microtome by coating a base blade substrate, for example, sapphire, with an approximately 5 to 50 nm thick layer of diamond by plasma-induced chemical vapor-phase deposition and subsequent heat treatment at 700 - 1300°C to expel adsorbed impurities in the diamond layer. Kitamaru et al. (US 4,980,021) further discloses etching the surface of the carbonaceous coating on the surface of the blade to provide beneficial surface roughness.

Bache et al. (US 5,032,243) describes a method of forming or modifying cutting edges of razor blades by subjecting a stack of stainless steel razor blades to ion bombardment from two ion sources located on opposite sides of a plane that lies within the stack and that is parallel to the major surfaces of the blades. A mechanically sharpened cutting edge is bombarded with ions from the two sources to build up a new edge, after which an electron beam evaporator is operated to vaporize the desired coating material or component thereof where the coating is a compound, and operation of the ion sources is continued. After deposition is commenced the sputter removal rate due to the ion sources should be less than the deposition rate and the ion sources are operated to ensure the deposition.

Hahn (US 5,048,191) describes a process for forming a razor blade by providing a ceramic substrate, mechanically abrading an edge of the substrate to form a sharp edge with facets that have an included angle of less than 30 degrees, thermally processing the mechanically abraded edge to reduce surface raggedness and subsurface defects, and sputter-sharpening the sharpened edge to provide supplemental facets having an included angle of more than 40 degrees to define a tip radius of less than 500 Å.

Kramer (US 5,121,660) describes a process for forming a razor blade that includes providing a polycrystalline ceramic substrate having a grain size less than 2 µm, mechanically abrading an edge of the substrate to form a sharpened edge having an included angle less than 20 degrees, and sputter-etching the sharpened edge to reduce the tip radius to less than 300 Å, forming thereby a cutting edge.

deJuan, Jr. et al. (US 5,317,938) describe a method for making a microsurgical cutter from a flat planar substrate. A photoresist mask layer is formed on the top surface of the substrate in a pattern of the microsurgical instrument and the top surface of the substrate is etched isotropically through to the bottom surface to form a cutting edge portion, with the cutting edge portion having a configuration corresponding to the edge portion of the mask layer. Semiconductor materials such as silicon, silicon carbide, sapphire and diamond can be used for the substrate.

Knudsen et al. (US 5,724,868) describe a method for making a knife with improved cutting performance. A steel knife blade blank is coated with TiN, Ti(CN) or (TiAl)N by a cathodic arc process using linear deposition sources with simultaneous heating and rotation of the blade blank relative to the deposition sources. The blade edge of the blank can be sharpened or unsharpened prior to the deposition of the coating. If the blank is unsharpened prior to deposition, it is thereafter sharpened, preferably on one side only, by conventional procedures using abrasive grinding and a final stropping of the blade.

Decker et al. (US 5,799,549) describe improved razor blades and processes for making sharp and durable cutting edges by hard carbon coating the sharpened edge of the blade substrates with amorphous diamond. The substrate can be mechanically honed and there is no interlayer between the substrate and the diamond coating. The coating imparts stiffness and rigidity to a thin blade while maintaining a high aspect ratio.

Marcus et al. (US 5,842,387) disclose knife blades having "ultra-sharp" cutting edges, which are fabricated from wafers of monocrystalline silicon. First, the wafer is covered with an etchant masking layer over an elongated ridge. Then the wafer is etched to undercut the mask and to shape ridge sidewalls converging toward the ridge tip. A sharp ridge apex is provided using an oxide forming/oxide stripping process. Blades having excellent sharpness are obtained, however, the oxide forming/oxide stripping cycles of the process are time consuming. Further, the extremely sharp blade edges are relatively fragile and, in many applications, it is preferable to dull the edges and further strengthen the edges by the addition of one or more protective layers by, e.g., RF sputtering. In addition, blades exhibiting double bevels are difficult and expensive to fabricate with this teaching.

Consequently, there continues to be a need for sharper and more durable edge on cutting instruments, especially for precision surgery. Indeed, there remains an unresolved need in the industry for an economical cutting instrument that provides an atomically sharp cutting edge and blade tip.

In this setting it would be desirable to produce limited reuse or disposable, single- or double-beveled cutting instruments, which exhibit exceptional sharpness, excellent wear resistance, and minimal blade resistive forces, and a method of manufacturing the reusable or disposable instrument for use in microsurgical procedures. Furthermore, it would be desirable to provide an instrument with a continuous cutting edge. Moreover, it would be desirable to manufacture such a cutting instrument from material that is biocompatible for use in surgical instruments. It would also be desirable to provide such an instrument and a method of making the instrument economically.

### SUMMARY OF THE INVENTION

The present invention, which is defined by claims 1, 8 and 13, provides a blade having an atomically sharp cutting edge made of a hard durable material. The present invention uses focused ion beam (FIB) milling technology to "atomically mill" an atomically sharp edge to the blade of a cutting instrument, i.e., the edge is sharp on a sub-micron scale and can have a radius of curvature on the order of about 1Å to about 300Å.

FIB technology has been developed to "ion mill" or "etch" highly precise integrated circuit patterns into semiconductor materials. FIB conditions and techniques have been described in US 5,482,802, US 5,658,470, US 5,690,784, US 5,744,400, US 5,840,859, US 5,852,297 and US 5,945,677.

Devices in accord with the present invention can have a single beveled cutting edge or a double beveled cutting edge.

Other aspects and embodiments of the invention are discussed below. Additional objects and advantages of the present invention will be apparent from the drawings and descriptions that follow.

### BRIEF DESCRIPTION OF THE DRAWINGS

For a fuller understanding of the nature and desired objects of the present invention, reference is made to the following detailed description taken in conjunction with the accompanying drawing figures wherein like reference character denote corresponding parts throughout the several views and wherein:
FIG. 1 is a diagrammatic view of the cutting edge of a blank for a cutting blade after a coating has been deposited but before focused ion milling of a new cutting edge, in accord with one embodiment of the present invention. FIG. 2 is a diagrammatic view of the cutting edge of a blank for a cutting blade that has been coated and focused ion milled to form a new cutting edge, in accord with one embodiment of the present invention.
FIG. 3A is an embodiment of a wafer dicing process for producing rough blanks.
FIG. 3B shows an embodiment of blank diagramatically subjected to focused ion beam milling, wherein the atomically polished side of the blank does not form part of the cutting edge.
FIG. 3C shows an alternate embodiment of a blank diagramatically subjected to focused ion beam milling, wherein the atomically polished side of the blank forms part of the cutting edge.
FIG. 4A is a digital microphotograph illustrating a conventionally sharpened edge having a typical microscopic edge defect.
FIGs. 4B-4E are digital microphotographs illustrating conventionally sharpened edges having microscopic rollover at the edge and/or microscopic debris.
FIG. 5A is a plan view illustrating an embodiment of cutting edge blanks useful for preparing an atomically sharp cutting edge in accord with the present invention.
FIG. 5B is a cross sectional view taken at 5B-5B in FIG. 5A.
FIG. 6 is a digital microphotograph illustrating a conventionally sharpened edge wherein a portion thereof was further sharpened by focused ion beam milling in accord with the present invention, thereby showing the dramatic results of focus ion beam milling for an atomically sharp cutting edge.
FIG. 7 illustrates a sharpened cutting edge blank in accord with the present invention attached to a substrate.

### DETAILED DESCRIPTION OF THE INVENTION INCLUDING PREFERRED EMBODIMENTS

Atomically sharp cutting blades in accord with the present invention include a cutting edge portion comprised of a hard material having a sharpened edge formed by focused ion beam (FIB) milling of the sharpened edge. Suitable hard materials for the practice of the present invention Si, Al₂O₃, TiN, AlTiN, SiC, SiN, molybdenum disulfide (MoS₂), amorphous carbon, diamond-like carbon, zircon, and similar materials that are removable by a focused ion beam. The edge portion of hard material can be supported on a substrate, typically of a softer, more robust material. Alternatively, the hard material can be formed into a wafer on which a sharpened edge is formed by a focused ion beam.

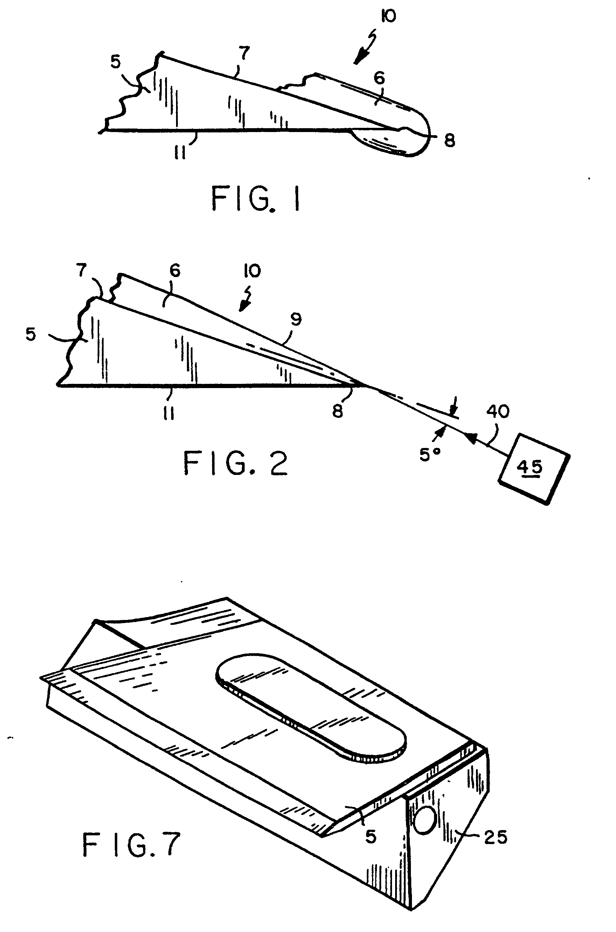

In a one embodiment of the present invention (FIG. 1), a metal cutting edge blank 5 is formed by any method, e.g., powered cast metallurgy, forging, coming, electric discharge machining, micro-machining, photo-etching, or the like. The blank 5, preferably, is formed with at least one tapered cutting edge 7. Thus, the cutting edge 7 is substantially less thick than the rest of the blank 5. The blank 5 is made of any thickness suitable for the cutting tool or surgical instrument 10 that is desired.

The formed cutting edge blank 5 is cleaned and a coating 6 of a material that is substantially harder than the material of the blank 5 is applied thereto, for example, by chemical vapor deposition, sputtering or ion-assisted deposition. These processes, which are well known to those of ordinary skill in the art, typically utilize high vacuums in which the vacuum pressure is less than 10⁻² Torr, e.g., 10⁻³, 10⁻⁴, etc. The hard material coating 6 can be formed in the presence of suitable gaseous elements applied by the vacuum deposition process. Indeed, it also is known to those skilled in the art that the presence of certain gasses assists in the adhesion of the coating 6 to the blank 5. Suitable hard materials for the deposited coating 6 are Si, Al₂O₃, ALTiN, TiN, SiC, SiN, MoS₂, amorphous carbon, diamond-like carbon, zircon, and similar such materials.

The coating 6 is applied so that a substantial layer of the harder material continuously covers at least one side of the cutting edge 7 of the blank 5. Preferably, the coating 6 forms a continuous layer at the cutting edge 7. Coatings 6 as thick as 30,000 Å have been applied; but, usually, it is more economical to apply a coating that is on the order of about 500 Å or less. Indeed, in the preferred embodiment, the thickness for the coating 6 after focused ion milling is from about 100 Å to about 500 Å, more preferably at least about 200 Å.

In an application in which a coating 6 is desired on only one side, i.e., for the cutting edge 7 of a single-beveled cutting instrument 10, the coating process typically deposits excess coating material 6 on the base surface 11, which must be removed. Indeed, it is desirable to process the portion of the blade that was not intended to be coated (i.e., the base surface 11), e.g., by non-focused ion milling or ion etching, to remove the undesirable excess hard material 6. Thereafter, the blank 5 is sharpened by focused ion beam (FIB) milling to create a new sharpened edge 9 having an atomically sharp edge. For double-beveled cutting instruments, on which both sides of the blank are tapered and coated with a harder material, each coated side of the blank is milled by a focused ion beam to create a new sharpened end having an atomically sharp double-beveled edge.

The focused ion beam removes portions of the coating 6 from the edge 7 and reestablishes a new, "atomically sharp" cutting edge 9 (FIG. 2) that is displaced by the remaining, i.e., non-milled, thickness of the coating 6 from the original cutting edge 7 of the blank 5. U.S. Patent Number 5,945,677, describes a process for providing a focussed ion beam (FIB) that can be used for nanolithography. Such a FIB can be focussed at an angle on the cutting edge of the coated blank 5 for removing hard material 6 to form a new atomically sharp cutting edge 9.

Focused ion beam milling, as described above, is performed with specialized equipment manufactured by Micrion Division of FEI or the like. The focused ion beam 40, which forms the working energy source, preferably is derived from an electrically excited liquid gallium source 45; however, other ion sources 45 that are known to those skilled in the art can be used it within the scope of the claims. The source 45 emits a beam of gallium ions 40 that are focused to a desired diameter. The focused beam 40 is spatially limited, preferably, to about 5 nm in diameter. Larger focused beam diameters, e.g., 10 nm, however, also can be used with satisfactory results. The level of energy that is required to mill a sharp cutting edge 9 on a blade blank 5 ranges from about 30 pA for a 5 nm diameter beam to about 100 pA for a 10 nm diameter beam. As in any milling process, the object of this processing step is to remove (1) some or all specific areas of the harder coating 6 along the desired cutting edge 9 and (2) in the plane of the base surface 11, or, for double-beveled cutting edges, removing the hard coating along the desired cutting edges.

The focused ion beam 40 cuts like an atomic milling machine, allowing stress-free, in-situ sectioning to form an atomically-milled cutting edge 9.

The focused ion beam 40 is directed at the coating 6 on the blank 5 at an angle within a few degrees of the desired final angle of the sharpened cutting edge 9. Typically, the beam 40 is directed toward the cutting edge 9 of the blank 5 at an angle of about five (5) degrees greater than the reference to a plane parallel to the major surface of the blank cutting edge 9. The processes of the present invention provide a cutting edge 9 having a high quality of the surface finish and the repeatability of the dimensional tolerance of the formed product. It is preferred that the dimensional tolerance is accurate to at least ± 0.3 microns, or less. Moreover, a radius of curvature of the cutting edge of less than about 300 Å can be provided, preferably less than about 100 Å, more preferably less than about 10 Å. Furthermore, by making the cutting edge in accord with the present invention, microscopic debris is avoided at the cutting edge.

The cutting edge blank 5 having the new atomically sharpened edge is finally joined to a support substrate (FIG. 7), which can be, e.g., metal or plastic, which takes practically any shape or form and provides structural support, strength, and shatter resistance to the resulting cutting edge 9. as illustrated in FIG. 7, a double-beveled, atomically sharpened blank is mounted to substrate 25 by means of adhesive (not shown). The substrate can be made of any suitable material such as a metal or a plastic.

In a second embodiment, cutting edge blanks 60 are made from a wafer-like sheet 65 of metallic silicon, ceramic, glass, Al₂O₃, ALTiN, TiN, SiC, SiN, MoS₂, amorphous carbon, diamond-like carbon, zircon, and similar such hard material, cutting edge blanks having a variety of shapes can be fashioned from a sheet 65, by micro-machining, non-focused ion beam milling, or etching (FIGS. 5a and 5b), which processes are well known to those of ordinary skill in the art. In this embodiment, a plurality of blanks 60 is first rough formed by the said processes. The cutting edge blanks 60 are chemically etched to provide a cutting edge 63 and are rough sawn to provide a back edge. Then, the cutting edge 63 is sharpened by milling with a focused ion beam, as described above, as necessary on one or two sides, to provide the desired atomically sharp cutting edge. This embodiment can provide sharpened cutting edges relatively quickly. The blanks with FIB milled sharpened edges can be mounted on supports (e.g., see FIG 7) to provide cutting instruments.

Rectangular blanks 61, which are made by micro-machining, etching or non-focused beam milling, are the preferred shape; however, e.g., circular, elliptical, triangular, and polygonal shapes are within the teaching of this invention. To form the rectangular blanks 61, micro-machining, etching or non-focused beam milling is performed in such a manner as to define the perimeter of the rectangular blank 61. The taper angle 62 of the cutting edge 63 of the rough blank 61 typically is between about 30 and about 60 degrees, preferably about 36.8 degrees as shown in FIG. 5b. The rectangular blank 61 is then milled with a focused ion beam to create an atomically sharp cutting edge at 63. The individual cutting edge blanks 60 can be, e.g., halved 69 from the rectangular blanks 61. As an example, in Figure 5a, a rectangular blank 61 is halved 69 to produce a pair of cutting edged blanks 60 with three atomically sharp cutting edges 63. The cutting edge can be provided in any shape. A support substrate, e.g., of metal plastic, glass, etc., is then laminated to the cutting edged blank 60 to provide structural support, strength, and shatter resistance.

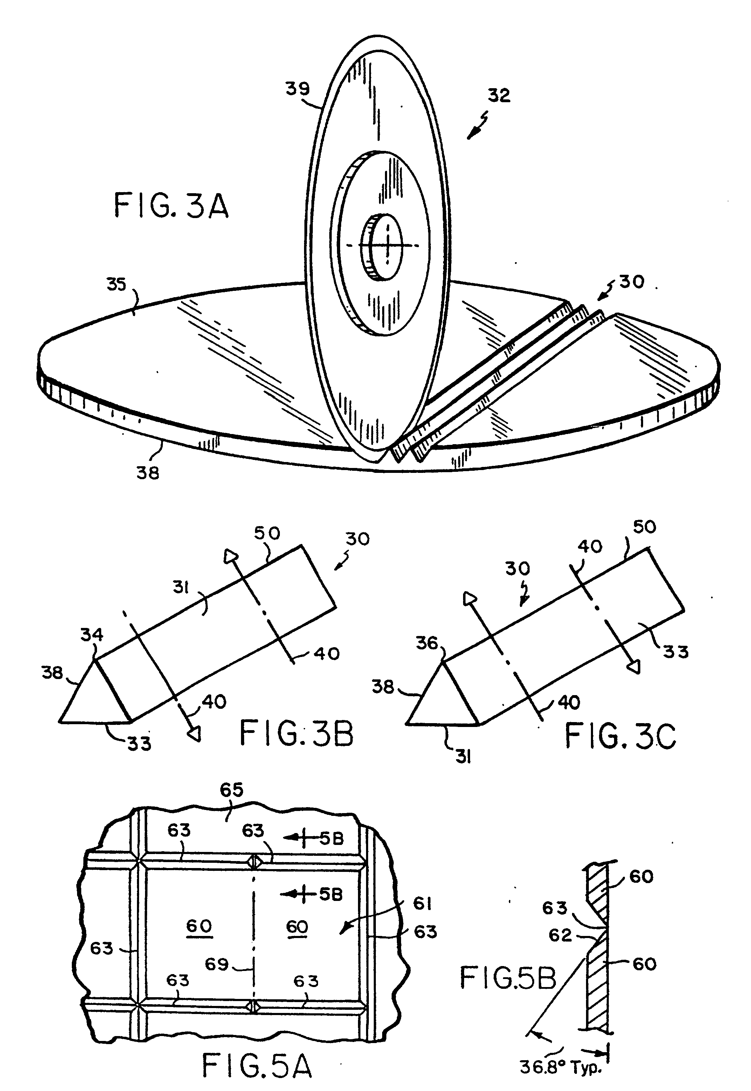

In another embodiment (FIG. 3a), a plurality of blade cutting edge blanks 30 are fabricated from wafers 35 of, e.g., silicon, ceramic, glass, Al₂O₃, ALTiN, TiN, SiC, SiN, MoS₂, amorphous carbon, diamond-like carbon, zircon, and similar such hard material, preferably materials that are readily available in the form of wafers from the semiconductor industry. Preferably, the wafer 35 thickness is between about 100 µm and about 1000 µm. More preferably, at least one side 38 of the wafer 35 is polished to an atomic finish, which feature is also common in the semiconductor industry.

Initially, a wafer-dicing saw 32 is used on the wafer 35 to provide a rough geometry for the cutting edge blank 30. Indeed, the object of the dicing operation is to form, or create, a series of elongated cutting edge blanks 30, e.g., triangular prism shaped components, across the surface of the wafer 35. Before dicing, the wafer 35 is mounted securely in a fixture and, then, a cutting saw 32 systematically produces a plurality of blanks 30. The cutting saw 32 is equipped with a specially prepared blade 39 (e.g., diamond, silicon carbide, or the like) with a cutting face of about 100 µm. The specially prepared blade 39 of the cutting saw 32 is capable of forming precision cut bevels in the wafer 35 wherein the peaks being formed have an included angle ranging preferably from about 10 to about 90 degrees; however, the cutting media of the saw 32 is selected so that it does not produce chips greater than 0.3 µm in dimension.

After the sawed wafer 35 is cut into cutting edge blanks 30, it is cleaned, e.g. by ultrasonic cleaning, plasma, high-pressure de-ionized water, and the like, to remove all debris and cutting solvent that may have contaminated the surface. The cutting edge blanks 30, then are loaded into a vacuum chamber of a focused ion beam mill. The chamber is exhausted to a vacuum pressure of about 10⁻⁷ Torr. Then a focused ion beam 40 is directed along at least one side 31, 33 toward the apex 34 of the blade blank 30 (FIG. 3B).

Cutting edge blanks can also be made by chemical etching techniques, which are well known to those skilled in the art. For example, a silicon wafer is provided with a photoresist mask, which is resistant to the etching solution being used. The mask consists of longitudinal strips of resist located at the position of top blade edge of the cutting edge blank, i.e., the apex of the cutting edge blank having a height perpendicular to the plane of the wafer. For an etching depth in the wafer of 150 microns, it is necessary to provide at least a one micron wide strip of resist along the top blade edge to prevent under etching and damage to the edge structure. The final edge is shaped by the FIB.

Depending on the desired final use and/or the ultimate radius of curvature, i.e., edge sharpness, the focus ion beam 40 can be directed at the blade blank 30 from in front of the leading edge 50 or from behind the leading edge 50 (FIG. 3B). Ultimate sharpness is created when the focused ion beam source 45 preferably is applied to the cutting edge 50 from behind the leading edge of the cutting edge blank 30. Sharpened edges having included angles of from about 10 to about 70° are preferred. Thus, FIB angles of from about 5 to about 70° typically are used.

Preferably, an edge having polished side 38 adjacent thereto is used and the FIB is directed from an opposite side 31 to provide the atomically sharpened single bevel edge.

Once the desired atomically sharp edge 50 has been produced on the cutting edge blank 30, the base of the cutting edge blank 30 (opposite the sharpened edge, e.g., side 33) is fixedly attached to a support substrate (e.g., see FIG. 7), that is made, e.g., of metal, plastic, glass, ceramic or the like by means of, e.g., solder, epoxy, brazing, staking, crimping, adhesives, friction fitting or eutectic bonding. The support substrate facilitates ultimately attaching the mounted, atomically sharp cutting edge blank 30 to any desired cutting instrument or tool body. It also is possible to practice this invention by attaching the sharpened blade blank 30 directly to the cutting instrument or tool body.

For a double beveled cutting edge, the FIB is directed from both sides of the cutting edge.

Using the focused ion beam mechanism in the transverse, high current mode, it is possible to sculpt various edge geometries by a process referred to as "beam shaping". The focused ion beam for "beam shaping" has a preferred beam diameter of at least about 10 nm.

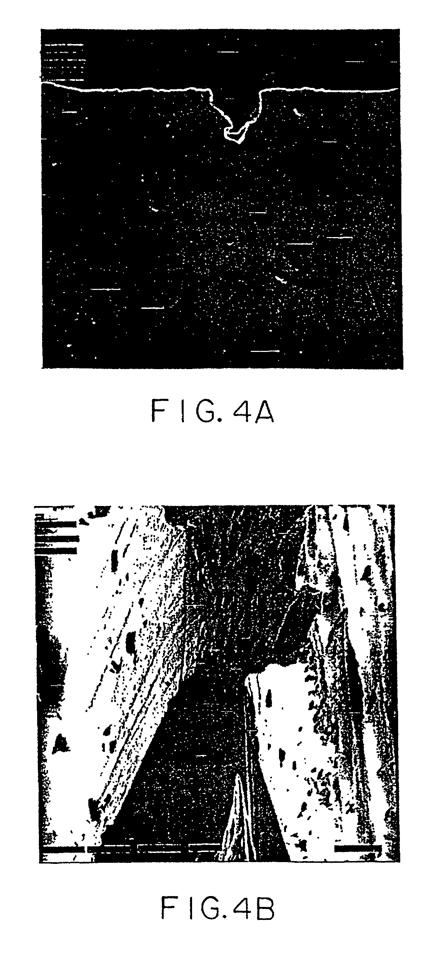

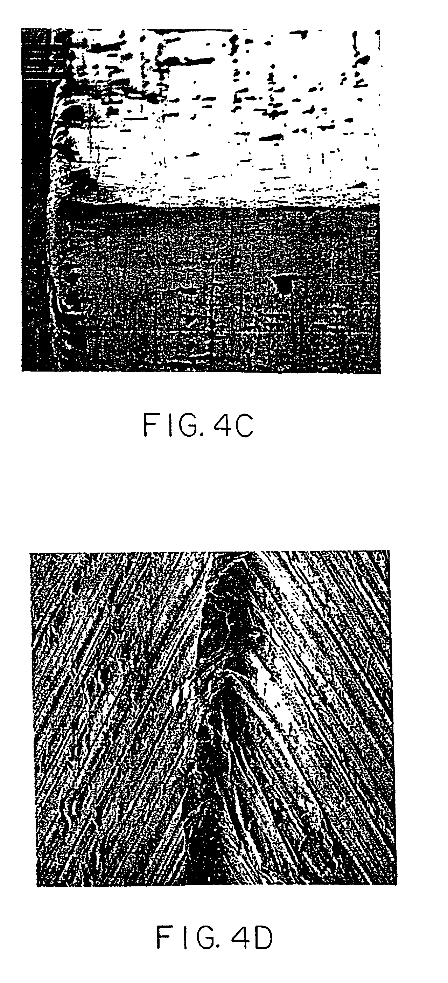

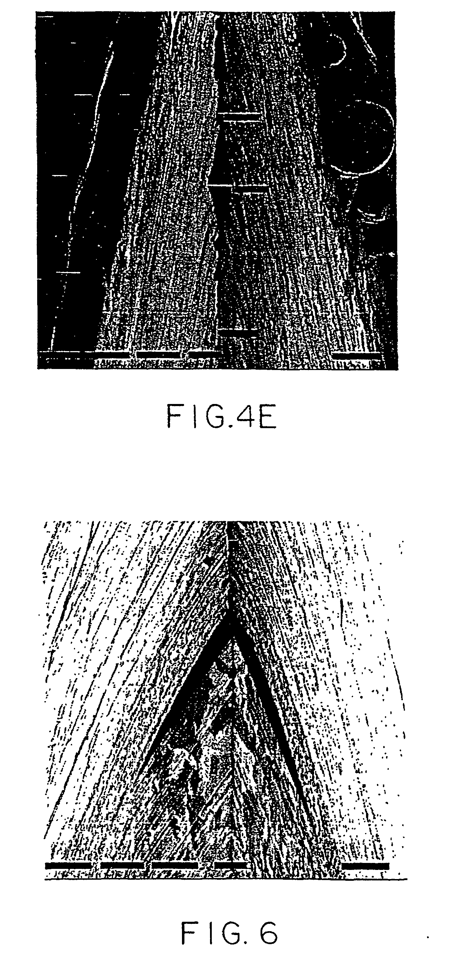

FIGs. 4A-4E illustrate the edges of prior art blades mechanically sharpened and honed to provide extreme sharpness for microsurgical instruments. FIG. 4A shows a typical defect in the sharpened edge. FIGs. 4B-4E show typical rollover of metal at the sharpened edge and microscopic debris left by the mechanical sharpening and honing processes.

FIG. 6 graphically demonstrates the dramatic improvement in sharpness resulting from focused ion beam milling. FIG. 6 depicts two areas on a blade edge. The first area 52 has been sharpened with conventional abraded grinding and honing of the edge. It has microscopic debris around the sharpened edge and the edge, itself, shows rollovers. The second area 54 has been milled with a focused ion beam 40 in accord with the present invention. Note the surprisingly clean surfaces around the sharpened edge and the clean atomically sharp edge.

## Claims

1. A method for making an atomically sharp cutting edge (9, 50) for a cutting instrument, said method comprising:
providing a blank (5, 30, 61) made of a metal material and having a major surface and a tapered edge at one end of the major surface;
depositing on a portion of the major surface at the tapered edge a continuous layer of a second material that is harder than the metal; and **characterized by** milling the layer of the second material with a focussed ion beam (40) to form an atomically sharp cutting edge (9, 50)

2. A method as set forth in claim 1, wherein the layer of second material is deposited in a thickness from about 100 to about 500 Å, preferably in a thickness at least about 200 Å.

3. A method as set forth in claim 1 or 2, wherein the second material is selected from a group consisting of silicon, ceramic, glass, Al₂O₃, AlTiN, TiN, SiC, SiN, MoS₂, amorphous carbon, diamond-like carbon and zircon.

4. A method as set forth in any of claims 1 to 3
wherein the layer is milled at an acute angle to a plane parallel to said major surface by a focused ion beam (40) to provide said blank with a continuous, atomically sharp cutting edge (9, 50).

5. A method as set forth in claim 4, wherein a support substrate 25 is provided to which the metal blank is attached.

6. A method as set forth in claim 4 or 5, wherein the layer of second material is from about 100 to about 500 Å thick and/or the coating layer is at least about 200 Å thick.

7. A method as set forth in one of claims 4 to 6, wherein the second material comprises a material selected from a group consisting of silicon, ceramic, glass, Al₂O₃, AlTiN, TiN, SiC, SiN, MoS₂, amorphous carbon, diamond-like carbon and zircon.

8. A method for making an atomically sharp cutting edge for a cutting instrument, said method comprising:
providing a blade blank (5, 30, 61) having a major surface and an edge (7) at one end thereof, **characterized by**
milling said edge at an acute angle to a plane parallel to said major surface using a focused ion beam (40) to provide said blank with a continuous, atomically sharp cutting edge (9).

9. A method as set forth in claim 8, wherein the blank is a wafer (35, 65) comprising a material selected from a group consisting of silicon, ceramic, glass, Al₂O₃, AlTiN, TiN, SiC, SiN, MoS₂, amorphous carbon, diamond-like carbon and zircon and/or wherein the blank is formed with a thickness of from about 100 µm to about 1000 µm.

10. A method as set forth in one of claims 1 to 9 wherein the cutting edge (9, 50) is formed with a radius of curvature that is less than about 300 Å, preferably less than about 100 Å, further preferably less than about 10 Å.

11. A method as set forth in claim 8 or 9, wherein the cutting edge (7) of the blade blank (5) has a blade edge of less than about 1 micron before sharpening.

12. A method as set forth in one of claims 4 to 7 or 11, wherein the cutting instrument further comprises a support substrate (25) for attaching thereto said blade blank (5) with a continuous, atomically sharp cutting edge (9), wherein the support substrate is preferably selected from a group comprising metal, plastic, glass, or ceramic.

13. A method for producing an atomically sharp cutting edge (30, 61) for a cutting instrument, said method comprising the steps of:
providing a wafer (35, 65) of a material suitable for forming a cutting edge;
cutting the wafer to produce at least one blade blank having a triangular shaped cross section, said blade blank having a plurality of edges; **characterized by**
positioning the blade blank in a vacuum chamber;
exhausting the vacuum chamber to a desired pressure; and
milling an edge of the blade blank with a focused ion beam (40) to provide an atomically sharp cutting edge (50) on the blade blank.

14. A method as set forth in claim 13, further comprising attaching the atomically sharpen blade blank to a cutting instrument substrate and/or wherein the wafer (35, 65) comprises a material selected from a group consisting of silicon, ceramic, glass, Al₂O₃, AlTiN, TiN, SiC, SiN, MoS₂, amorphous carbon, diamond-like carbon and zircon and/or wherein the wafer is formed about 100 to about 1000 microns thick.

15. A method as set forth in claim 13 or 14, comprising cutting the wafer (35, 65) an angle to the surface of the wafer ranging between about 5 and about 70 degrees and/or comprising providing the wafer with at least one atomically polished surface and/or comprising providing the focus ion beam with a diameter of 5 nm or comprising providing the focus ion beam with a diameter of 10 nm.

16. A method as set forth in one of claims 1 to 3 or 8 to 10 or 13 to 15, wherein the cutting edge is formed with a single beveled edge or a double-beveled edge.

## Patentansprüche

1. Verfahren zur Herstellung einer atomar scharfen Schneidkante für ein Schneidinstrument, wobei das Verfahren aufweist:
Bereitstellen eines aus einem Metall hergestellten Rohlings (5, 30, 61), der eine Hauptseite besitzt, und eine spitz zulaufende Kante an einem Ende der Hauptseite;
Aufbringen einer kontinuierlichen Schicht eines zweiten Materials, das härter als das Metall ist, auf einem Teil der Hauptseite an der spitz zulaufenden Kante; und gekennnzeichnet durch
Abtragen der Schicht des zweiten Materials mit einem fokussierten Ionenstrahl (40), um eine atomar scharfe Schneidkante (9, 50) zu erzeugen.

2. Verfahren gemäß Anspruch 1, wobei die Schicht des zweiten Materials in einer Dicke von ungefähr 100 bis ungefähr 500 Å, vorzugsweise mit einer Dicke von wenigstens ungefähr 200 Å aufgebracht wird.

3. Verfahren gemäß Anspruch 1 oder 2, wobei das zweite Material aus einer Gruppe ausgewählt wird, die aus Silicium, Keramik, Glas, Al₂O₃, AlTiN, TiN, SiC, SiN, MoS₂, aus Aktivkohle, aus diamantähnlichem Kohlenstoff und Zirkon besteht.

4. Verfahren gemäß einem der Ansprüche 1 bis 3, wobei die Schicht in einem spitzen Winkel zu einer Ebene parallel zur Hauptseite mittels eines fokussierten Ionenstrahls (40) abgetragen wird, um den Rohling mit einer kontinuierlichen, atomar scharfen Schneidkante (9, 50) zu versehen.

5. Verfahren gemäß Anspruch 4, wobei ein Trägersubstrat (25) vorgesehen ist, auf dem der Metallrohling angebracht ist.

6. Verfahren gemäß Anspruch 4 oder 5, wobei die Schicht des zweiten Materials eine Dicke von ungefähr 100 bis ungefähr 500 Å und/oder die Beschichtungsschicht eine Dicke von wenigstens ungefähr 200 Å besitzt.

7. Verfahren gemäß einem der Ansprüche 4 bis 6, wobei das zweite Material ein Material aufweist, das aus einer Gruppe ausgewählt ist, die aus Silicium, Keramik, Glas, Al₂O₃, AlTiN, TiN, SiC, SiN, MoS₂, aus Aktivkohle, aus diamantähnlichem Kohlenstoff und Zirkon besteht.

8. Verfahren zur Herstellung einer atomar scharfen Schneidkante für ein Schneidinstrument, wobei das Verfahren aufweist:
Bereitstellen eines Klingenrohlings (5, 30, 61), der eine Hauptseite und an einem Ende hiervon eine Kante (7) besitzt, **gekennzeichnet durch**,
Abtragen der Kante in einem spitzen Winkel zu einer Ebene parallel zur Hauptseite unter Verwendung eines fokussierten Ionenstrahls (40), um den Rohling mit einer kontinuierlichen atomar scharfen Schneidkante (9) bereitzustellen.

9. Verfahren gemäß Anspruch 8, wobei der Rohling ein Wafer (35, 65) ist, der ein Material aufweist, das aus einer Gruppe ausgewählt ist, die aus die aus Silicium, Keramik, Glas, Al₂O₃, AlTiN, TiN, SiC, SiN, MoS₂, aus Aktivkohle, aus diamantähnlichem Kohlenstoff und Zirkon besteht, und/oder wobei der Rohling mit einer Dicke von ungefähr 100 µm bis ungefähr 1.000 µm ausgebildet ist.

10. Verfahren gemäß einem der Ansprüche 1 bis 9, wobei die Schneidkante (9, 50) mit einem Krümmungsradius ausgebildet ist, der geringer als ungefähr 300 Å, vorzugsweise geringer als ungefähr 100 Å, noch weiter vorzugsweise geringer als 10 Å ist.

11. Verfahren gemäß Anspruch 8 oder 9, wobei die Schneidkante (7) des Klingenrohlings (5) vor dem Schärfen eine Klingenkante von weniger als ungefähr 1 µm besitzt.

12. Verfahren gemäß einem der Ansprüche 4 bis 7 oder 11, wobei das Schneidinstrument des weiteren ein Trägersubstrat (25) aufweist, um daran den Klingenrohling (5) mit einer kontinuierlichen, atomar scharfen Schneidkante (9) zu befestigen, wobei das Trägersubstrat vorzugsweise aus einer Gruppe ausgewählt ist, die Metall, Plastik, Glas oder Keramik aufweist.

13. Verfahren zur Herstellung einer atomar scharfen Schneidkante (30, 61) für ein Schneidinstrument, wobei das Verfahren die folgenden Schritte aufweist
Bereitstellen eines Wafers (35, 65) aus einem Material, das zur Erzeugung einer Schneidkante geeignet ist;
Schneiden des Wafers zur Herstellung von wenigstens einem Klingenrohling, der einen dreieckigen Querschnitt besitzt, wobei der Klingenrohling eine Mehrzahl von Kanten besitzt, **gekennzeichnet durch**
Positionieren des Klingenrohlings in einer Vakuumkammer;
Evakuieren der Vakuumkammer auf einen gewünschten Druck; und
Bearbeiten einer Kante des Klingenrohlings mit einem fokussierten Ionenstrahl (40) zum Bereitstellen einer atomar scharfen Schneidkante (50) auf dem Klingenrohling.

14. Verfahren gemäß Anspruch 13, des weiteren aufweisend den Schritt zu Befestigung des atomar scharfen Klingenrohlings an einem Schneidinstrumentensubstrat und/oder wobei der Wafer (35, 65) ein Material aufweist, das ausgewählt ist aus einer Gruppe, die aus die aus Silicium, Keramik, Glas, Al₂O₃, AlTiN, TiN, SiC, SiN, MoS₂, aus Aktivkohle, aus diamantähnlichem Kohlenstoff und Zirkon besteht und/oder wobei der Wafer mit einer Dicke von ungefähr 100 bis ungefähr 1.000 µm ausgebildet ist.

15. Verfahren gemäß Anspruch 13 oder 14, aufweisend den Schritt des Schneidens des Wafers (35, 65) in einem Winkel zur Oberfläche des Wafers, der im Bereich zwischen ungefähr 5 bis ungefähr 70° liegt und/oder den Schritt aufweisend, den Wafer mit wenigstens einer atomar polierten Oberfläche zu versehen, und/oder den Schritt aufweisend, den fokussierten Ionenstrahl mit einem Durchmesser von 5 nm bereitzustellen oder den Schritt aufweisend, den fokussierten Ionenstrahl mit einem Durchmesser von 10 nm vorzusehen.

16. Verfahren gemäß einem der Ansprüche 1 bis 3 oder 8 bis 10 oder 13 bis 15, wobei die Schneidkante mit einer einzigen abgeschrägten Kante oder einer doppelt abgeschrägten Kante ausgebildet ist.

## Revendications

1. Un procédé de fabrication d'une arête de coupe aiguisée atomiquement (9,50) pour un instrument de coupe, ledit procédé comprenant :
- la prise d'une ébauche (5,30,61) faite d'un matériau métallique et ayant une surface principale et un bord biseauté à une extrémité de la surface principale ;
- le dépôt sur une partie de la surface principale au niveau du bord biseauté, d'une couche continue d'un second matériau qui est plus dur que le métal et
**caractérisé par**:
- l'abrasion de la couche de second matériau à l'aide d'un faisceau ionique focalisé (40) pour former une arête de coupe aiguisée atomiquement (9,50).

2. Un procédé selon la revendication 1, dans lequel la couche de second matériau est déposée selon une épaisseur d'environ 100 à environ 500 Å, de préférence à une épaisseur d'au moins environ 200 Å.

3. Un procédé selon la revendication 1 ou 2, dans lequel un second matériau est choisi dans le groupe consistant en silicone, céramique, verre, Al₂O₃, AlTiN, TiN, SiC, SiN, MoS₂, carbone amorphe, carbone de type diamant et zircone.

4. Un procédé selon l'une des revendications 1 à 3, dans lequel la couche est abrasée à un angle aigu par rapport à un plan parallèle à ladite surface principale par un faisceau ionique focalisé (40) afin de doter ladite ébauche d'une arête de coupe continue, aiguisée atomiquement (9,50).

5. Un procédé selon la revendication 4, dans lequel on utilise un substrat de support (25) sur lequel est fixée l'ébauche de métal.

6. Un procédé selon la revendication 4 ou 5, dans lequel la couche de second matériau a une épaisseur d'environ 100 à environ 500 Å et la couche de revêtement a une épaisseur d'au moins environ 200 Å.

7. Un procédé selon l'une quelconque des revendications 4 à 6, dans lequel le second matériau comprend un matériau choisi dans le groupe consistant en silicone, céramique, verre, Al₂O₃, AlTiN, TiN, SiC, SiN, MoS₂, carbone amorphe, carbone de type diamant et zircone.

8. Un procédé de fabrication d'une arête de coupe aigiùsée atomiquement pour un instrument de coupe, ledit procédé comprenant :
la prise d'une ébauche de lame (5, 30, 61) comportant une surface principale et un bord à l'une de ses extrémités, **caractérisé par** l'abrasion dudit bord à un angle aigu par rapport à un plan parallèle à ladite surface principale en utilisant un faisceau ionique focalisé (40) pour doter ladite ébauche d'une arête de coupe continue, aiguisée atomiquement (9).

9. Un procédé selon la revendication 8, dans lequel l'ébauche est une tranche (35, 65) comprenant un matériau choisi dans le groupe consistant en silicium, céramique, verre, Al₂O₃, AITiN, TiN, SiC, SiN, MoS₂, carbone amorphe, carbone de type diamant et zircone et/ou dans lequel l'ébauche est formée avec une épaisseur d'environ 100 µm à environ 1000 µm.

10. Un procédé selon l'une quelconque des revendications 1 à 9, dans lequel l'arête de coupe (9,50) est formée avec un rayon de courbure qui est inférieur à environ 300 Å, de préférence inférieur à environ 100 Å et tout particulièrement de préférence inférienr à environ 10 Å.

11. Un procédé selon la revendication 8 ou 9, dans lequel l'arête de coupe (7) de l'ébauche de lame (5) comporte une arête de lame inférieure à environ 1 micron avant aiguisage.

12. Un procédé selon l'une quelconque des revendications 4 à 7, ou 11 dans lequel l'instrument de coupe comprend en outre un substrat support (25) pour fixer à celui-ci ladite ébauche de lame (5) dotée d'une arête de coupe continue, aiguisée atomiquement (9), dans lequel le substrat de support est de préférence choisi parmi le groupe comprenant les métaux, matière plastique, le verre ou la céramique.

13. Un procédé de fabrication d'une arête de coupe aiguisée atomiquement (30, 61) pour un instrument de coupe, ledit procédé comprenant les étapes dans lesquelles :
on prend une tranche (35,65) d'un matériau qui convient pour former une arête de coupe ;
on découpe la tranche pour obtenir au moins une ébauche de lame ayant une section transversale de forme triangulaire, ladite ébauche de lame ayant une pluralité d'arêtes ;
**caractérisé en ce que**
on positionne l'ébauche de lame dans une chambre à vide ;
on met la chambre à vide à la pression désirée, et
on abrase l'arête de l'ébauche de lame à l'aide d'un faisceau ionique focalisé (40) pour obtenir une arête de coupe aiguisée atomiquement (50) sur l'ébauche de lame.

14. Un procédé selon la revendication 13, comprenant en outre la fixation de l'ébauche de lame aiguisée atomiquement à un substrat d'instrument de coupe et/ou dans lequel la tranche (35,65) comprend un matériau choisi dans le groupe consistant en silicone, céramique, verre, Al₂O₃, AlTiN, TiN, SiC, SiN, MoS₂, carbone amorphe, carbone de type diamant, zircone et/ou dans lequel la tranche est conçue de manière à avoir une épaisseur d'environ 100 à environ 1000 microns.

15. Un procédé selon la revendication 13 ou 14 comprenant la découpe de la tranche (35, 65) selon un angle par rapport à la surface de la tranche compris entre environ 5 et environ 70 degrés et/ou comprenant :
que l'on dote la tranche d'au moins une surface polie atomiquemeot et/ou
que le faisceau ionique focalisé a un diamètre de 5 nm ou que le faisceau ionique focalisé a un diamètre de 10 nm.

16. Un procédé selon l'une quelconque des revendications 1 à 3, ou 8 à 10, ou 13 à 15, dans lequel l'arête de coupe est formée d'un bord à biseau simple ou d'un bord à biseau double.
